# EUROPÄISCHE PATENTSCHRIFT

(11) **EP 3 193 833 B1**
(45) Veröffentlichungstag und Bekanntmachung des Hinweises auf die Patenterteilung: **31.07.2019**
(21) Anmeldenummer: 15766050.7
(22) Anmeldetag: 15.09.2015
(51) Int. Cl.: A61K 9/08, A61K 9/19, A61K 33/10, A61K 33/14, A61K 33/42

(54) **DIALYSELÖSUNG**
DIALYSIS SOLUTION
SOLUTION DE DIALYSE

(30) Priorität: 18.09.2014 DE 102014013885
(43) Veröffentlichungstag der Anmeldung: 26.07.2017
(73) Patentinhaber: Fresenius Medical Care Deutschland GmbH, 61352 Bad Homburg (DE)
(72) Erfinder: HUPPERT, Jochen, 66132 Saarbrücken (DE); MATHIS, Pascal, 66359 Bous (DE); BERLICH, Robert, 66606 St. Wendel (DE)
(74) Vertreter: Herrmann, Uwe
(86) Internationale Anmeldenummer: PCT/EP2015/001845
(87) Internationale Veröffentlichungsnummer: WO 2016/041634

(56) Entgegenhaltungen:
- EP-A1- 2 206 504
- WO-A2-00/64456

## Beschreibung

Die vorliegende Erfindung betrifft eine Kombination aus Einzellösungen, die derart ausgebildet sind, dass sich nach ihrem Mischen eine Dialyselösung ausbilden, die Bicarbonat, Calcium sowie Phosphat enthält.

Calcium-haltige, bicarbonat-gepufferte Dialyselösungen enthalten üblicherweise Elektrolyte, Puffer und Glucose in physiologisch wirksamen Konzentrationen.

Aus dem Stand der Technik ist es bekannt, diese Lösungen in Form von Einzellösungen bereitzustellen, die in einem Doppelkammer-Beutel aufgenommen sind. Eine anwendungsfertige Dialyselösung wird durch das Mischen der beiden Kammerinhalte erhalten.

Ein Problem bei Dialyselösungen, die neben Calcium oder Magnesium auch Bicarbonat als Puffer enthalten, besteht darin, dass unter bestimmten Bedingungen, insbesondere bei einem vergleichsweise hohen pH-Wert und höheren Temperaturen schwerlösliche Carbonate gebildet werden können, was unerwünscht ist. Insbesondere ist eine pH-Wert Erhöhung bedingt durch einen Verlust an CO₂ durch Ausgasen für die Ausfällungsreaktionen verantwortlich.

Unter thermodynamischen Gesichtspunkten gibt es einen maximalen pH-Wert, bis zu dem die Dialyselösung stabil bleibt, d.h. bis zu dem die genannten Ausfällungen nicht auftreten. Steigt der pH-Wert der Dialyselösung unter Anwendungsbedingungen an, wie beispielsweise durch das Pumpen und Heizen an einer Dialysemaschine, kann ein metastabiler Zustand erreicht werden. Kollabiert dieser Zustand, fallen schwerlösliche Carbonate aus, was zu erheblichen Komplikationen bei der Behandlung führen kann. Dabei stellen Magnesiumcarbonat und Calciumcarbonat aufgrund der schlechten Löslichkeit im basischen Milieu die kritischsten Verbindungen dar.

Durch die Bereitstellung der Einzellösungen in einem Doppelkammerbeutel kann eine getrennte Lagerung von Calcium einerseits und Hydrogencarbonat andererseits und somit eine erhöhte Stabilität bei der Lagerung der Dialyselösung erreicht werden. Aus dem Stand der Technik ist es weiter bekannt, die Beutelfolie aus einer Hochbarrierefolie herzustellen, um dem Entweichen von CO₂ und somit der Erhöhung des pH-Wertes in der bicarbonathaltigen Einzellösung entgegenzuwirken.

Gleichwohl steigt trotzt dieser speziellen Verpackung der pH-Wert der bicarbonathaltigen Dialyselösung über die Lagerdauer an, was zur Folge hat, dass beim Mischen der beiden Einzellösungen der pH-Wert der Mischlösung, d.h. der fertigen Dialyselösung vor deren Gebrauch ebenfalls erhöht ist.

Um Ausfällungen beim Mischen oder bei der Anwendung an der Dialysemaschine zu vermeiden, muss sichergestellt sein, dass der pH-Wert der bicarbonathaltigen Dialyselösung sowie der pH-Wert der aus den Einzellösungen hergestellten Mischung in einem relativ engen Rahmen liegen.

Die EP 2 206 504 A1 offenbart stabile Lösungen zur Dialyse, die Bicarbonat, Calcium sowie Phosphat in einer Konzentration von unter 0,4 mmol/l enthalten.

Der vorliegenden Erfindung liegt somit die Aufgabe zugrunde, eine Dialyselösung der eingangs genannten Art dahingehend weiterzubilden, dass die Wahrscheinlichkeit für das Auftreten von Ausfällungen gegenüber bekannten Dialyselösungen verringert ist.

Diese Aufgabe wird durch eine Kombination an Einzellösungen mit den Merkmalen des Anspruchs 1 gelöst. Danach ist vorgesehen, dass die die Einzellösungen der Kombination derart ausgebildet sind, dass sich nach ihrem Mischen eine Dialyselösung bilden, die Phosphat mit einer Konzentration im Bereich von bis zu 0,4 mmol/l, vorzugsweise im Bereich von bis zu 0,375 mmol/ oder im Bereich von bis zu 0,25 mmol/l enthält und besonders bevorzugt im Bereich von bis zu 0,2 mmol/l enthält.

Die Anwesenheit von Phosphat behindert die Ausfällung von Calciumcarbonat, wobei vermutet wird, dass die Adsorption eines CaHPO₄(aq)-Komplexes an der Oberfläche von Calciumcarbonat zu einer Blockade der sogenannten "active crystal-growth sites" führt.

Durch die Anwesenheit von Phosphat in den in Anspruch 1 genannten Konzentrationsbereichen wird die Stabilität der Dialyselösung und von Einzellösungen, aus denen die Dialyselösung erhalten wird, signifikant erhöht, was darauf zurückzuführen ist, dass das Zusammenbrechen des metastabilen Bereichs verzögert oder komplett verhindert wird. So kann eine anwendungssichere Dialyselösung über den kompletten Lebenszyklus des Produktes, vorzugsweise über eine Dauer von 24 Monaten oder länger gewährleistet werden.

An dieser Stelle wird darauf hingewiesen, dass der Begriff "Dialyselösung" jede beliebige Lösung enthält, die im Rahmen der Dialyse zum Einsatz kommen kann. Darunter sind auch Konzentrate zu verstehen, die z.B. vor der Verwendung bei der Dialyse weiter verdünnt werden müssen, als auch anwendungsfertige Lösungen, die als solche im Rahmen der Dialyse verwendet werden können.

Des Weiteren wird darauf hingewiesen, dass der Begriff "Phosphat" das Phosphat-Anion als solches, wie auch Verbindungen umfasst, die dieses Ion enthalten, wie beispielsweise Salze oder Ester der Phosphorsäure. Vorzugsweise handelt es sich bei dem Phosphat um Orthophosphat.

Die Stabilisierung der Dialyselösung bzw. der unten genannten Einzellösungen erfolgt somit durch Zugabe von Phosphat, wodurch die obere pH-Wert Grenze, bei der eine Ausfällung von Calciumcarbonat erfolgt, weiter ins basische Milieu, d.h. zu höheren pH-Werten hin verschoben wird. Etwaige Ausfällungsreaktionen finden dann erst bei so hohen pH-Werten statt, die während der Dialysebehandlung bzw. während der Lagerung der Dialyselösung üblicherweise nicht erreicht werden.

Dies führt nicht nur zu einer erhöhten Lagerdauer, sondern auch zu einem erheblichen Sicherheitsgewinn bei Anwendung der Dialyselösung an einer Dialysemaschine.

Aufgrund der Tatsache, dass die Phosphat-Konzentration unterhalb physiologischer Konzentrationswerte liegt, wird die medizinische Wirksamkeit der Dialyselösung nicht beeinflusst.

In einer bevorzugten Ausgestaltung der Erfindung enthält die die Dialyselösung Phosphat in einem Bereich von 0,05 mmol/l bis 0,25 mmol/l, insbesondere bis 0,20 mmol/l.

Die Untergrenze der Konzentration von Phosphat in der Dialyselösung liegt vorzugsweise bei 0,05 mmol/l.

Die Dialyselösung enthält Elektrolyte und optional eine Kohlenhydratverbindung, bei der es sich vorzugsweise um Glucose handelt.

Gemäß der Erfindung ist vorgesehen, dass die Dialyselösung Natrium, ggf. Kalium, Calcium, Magnesium und Chlorid enthält. Die Nennung von Elementen im Rahmen der vorliegenden Erfindung bezieht sich auf deren Ionen.

Es kann vorgesehen sein, dass nur eine der Einzellösungen Phosphat enthält.

Das Phosphat kann grundsätzlich beispielsweise in Form von Dihydrogenphosphat vorliegen.

Eine weitere bevorzugte Ausgestaltung der Erfindung besteht darin, dass eine der Einzellösungen Calcium enthält und eine andere Einzellösung, die kein Calcium enthält, das Phosphat enthält. Vorzugsweise sind Calcium und das Phosphat getrennt voneinander gelagert. Darüber hinaus sind vorzugsweise Calcium und Hydrogencarbonat voneinander getrennt gelagert, d.h. liegen in unterschiedlichen Einzellösungen vor.

Phosphat und Hydrogencarbonat befinden sich in ein und derselben Einzellösung. Phosphat und Hydrogencarbonat sind in einer weiteren bevorzugten Ausgestaltung in keiner weiteren Einzellösung enthalten.

Eine erste Einzellösung enthält Calcium, Magnesium, Chlorid, ggf. Glucose und ggf. Kalium und eine zweite Einzellösung Natrium, Chlorid, Hydrogencarbonat und Phosphat enthält. Die erste Einzellösung enthält vorzugsweise weder Phosphat noch Hydrogencarbonat. Auch Natrium ist nur in der zweiten Einzellösung vorhanden. Die zweite Einzellösung enthält vorzugsweise weder Calcium noch Magnesium. Vorzugsweise enthält die zweite Einzellösung kein Kalium und/oder keine Glucose.

Calcium kann beispielsweise in einer Einzellösung in einem Konzentrationsbereich von 20 mmol/l bis 40 mmol/l und vorzugsweise bei 30 mmol/l vorliegen, Magnesium kann in einer Einzellösung in einem Konzentrationsbereich von 5 mmol/l bis 15 mmol/ und vorzugsweise bei 10 mmol/l vorliegen, Kalium kann beispielsweise in einer Einzellösung in einem Konzentrationsbereich von 0 mmol/l bis 100 mmol/l vorliegen, Natrium kann beispielsweise in einer Einzellösung in einem Konzentrationsbereich von 100 mmol/l bis 200 mmol/l und vorzugsweise bei 140 mmol/l bis 160 mmol/l und bevorzugt bei 147,5 mmol/l vorliegen, Hydrogencarbonat kann beispielsweise in einer Einzellösung in einem Konzentrationsbereich von 30 mmol/l bis 50 mmol/l und vorzugsweise bei 37 mmol/l vorliegen, Phosphat kann beispielsweise in einer Einzellösung in einem Konzentrationsbereich von 0,05 mmol/l bis 0,15 mmol/l und vorzugsweise bei 0,11 mmol/l vorliegen, Glucose kann beispielsweise in einer Einzellösung in einem Konzentrationsbereich von 100 mmol/l bis 120 mmol/l und vorzugsweise bei 111 mmol/l vorliegen und Chlorid kann in beiden Einzellösungen vorliegen.

Dabei kann die Chlorid-Konzentration in der Einzellösung, die Glucose enthält, in einem Konzentrationsbereich von 60 mmol/l bis 100 mmol/l und vorzugsweise bei 82 mmol/l liegen und in der Einzellösung, die das Phosphat enthält in einem Konzentrationsbereich von 100 mmol/l bis 120 mmol/l und vorzugsweise bei 110 mmol/l liegen

Die Mischlösung kann Natrium in einer Konzentration im Bereich von 120 mmol/l bis 160 mmol/ und vorzugsweise von 140 mmol/l aufweisen, Kalium im Bereich von 0 bis 4 mmol/l, Calcium im Bereich von 1,0 mmol/l bis 2,0 mmol/l und vorzugsweise von 1,5 mmol/l, Magnesium im Bereich von 0,2 mmol/l bis 0,8 mmol/l und vorzugsweise von 0,5 mmol/l, Chlorid im Bereich von 100 mmol/l bis 120 mmol/ und vorzugsweise 109 mmol/l, Hydrogencarbonat im Bereich von 30 mmol/l bis 40 mmol/ und vorzugsweise 35 mmol/l, Phosphat bzw. Dihydrogenphosphat im Bereich von 0,05 mmol/l bis 0,15 mmol/l und vorzugsweise 0,1 mmol/l und Glucose im Bereich von 0 mmol/l bis 7 mmol/l und vorzugsweise 5,6 mmol/l.

Eine mögliche Zusammensetzung der Einzellösung sowie der daraus hergestellten Mischlösung ergibt sich aus der folgenden Tabelle:

| [mmol/L] | Lösung A [250 ml] | Lösung B [4750 ml] | Mischlösung [5000 ml] |
|---|---|---|---|
| Natrium | 0 | 147,48 | 140 |
| Kallum | 0 | 0 | 0 |
| Calcium | 30 | 0 | 1,5 |
| Magneslum | 10 | 0 | 0,5 |
| Chlorid | 82 | 110,42 | 109 |
| Hydrogencarbonat | 0 | 36,95 | 35 |
| Dihydrogenphosphat | 0 | 0,105 | 0,1 |
| Glucose | 111 | 0 | 5,55 |
| pH-Wert (Freigabe) | 2,40 - 3,00 | 7,00 - 7,30 | 7,00 - 7,25 |
| pH-Wert (Ende Haltbarkeit) | 2,40 - 3,00 | 7,00 - 7,80 | 7,00 - 7,60 |

Grundsätzlich kann Kalium in der Lösung A beispielsweise in einer Konzentration von 40, 60 oder 80 mmol/l enthalten sein, in der Mischlösung ergeben sich dann Kalium-Konzentrationen von 2, 3 oder 4 mmol/l.

Wie aus dieser Tabelle ersichtlich, kann die Einzellösung (Lösung B), die das Phosphat enthält, in einem größeren Volumen vorliegen, als die Einzellösung (Lösung A), die Calcium enthält. In dem hier dargestellten Beispiel beträgt das Volumen der einen Einzellösung 4,75 I und das Volumen der anderen Einzellösung 0,25 I.

Denkbar ist es, dass die erste Einzellösung, die das Calcium enthält, kein Hydrogencarbonat und/oder kein Phosphat und/oder kein Natrium enthält.

Weiterhin ist es denkbar, dass die zweite Einzellösung, die das Phosphat enthält, kein Calcium und/oder kein Magnesium und/oder kein Kalium und/oder keine Glucose enthält.

Die eine Einzellösung weist einen pH-Wert im Bereich von 2,4 bis 3,0 auf. Bei dieser Einzellösung handelt es sich um diejenige, die Calcium, jedoch vorzugsweise kein Hydrogencarbonat enthält.

Eine andere, das Hydrogencarbonat enthaltende Einzellösung weist einen pH-Wert im Bereich von 7,0 bis 7,8 auf.

Der pH-Wert der Dialyselösung, die durch Mischen der Einzellösungen gewonnen wird, liegt vorzugsweise im Bereich von 7,0 bis 7,6.

Die vorliegende Erfindung betrifft des Weiteren einen Mehrkammerbeutel umfassend wenigstens zwei Kammern, wobei eine der Kammern eine Einzellösung der erfindungsgemäßen Kombination und eine andere Kammer eine weitere Einzellösung der erfindungsgemäßen Kombination aufweist.

In einer bevorzugten Ausgestaltung der Erfindung ist vorgesehen, dass der Mehrkammerbeutel wenigstens eine Naht oder ein sonstiges Trennmittel aufweist, die zwei Kammern voneinander trennt, wobei die Naht oder das sonstige Trennmittel vorzugsweise durch Druck auf eine der Kammern geöffnet werden kann.

Weitere Einzelheiten und Vorteile der Erfindung werden anhand eines in der Zeichnung dargestellten Ausführungsbeispiels näher erläutert.

Es zeigen:
- Figur 1:: pH-Wert Verlauf einer Dialyselösung über die Zeit bei pH-Wert-Erhöhung durch Ausgasen von CO₂,
- Figur 2:: pH-Wert Verlauf einer Dialyselösung ohne und mit Phosphat über die Zeit bei pH-Wert-Erhöhung durch Ausgasen von CO₂,
- Figur 3:: Abhängigkeit der Zeitdauer der Ausfällung von Calciumcarbonat sowie des pH-Wertes der Dialyselösung bei der Ausfällung von Calciumcarbonat von der Phosphat-Konzentration der Dialyselösung und
- Figur 4:: Abhängigkeit der Zeitdauer der Ausfällung von Calciumcarbonat von der Phosphat-Konzentration der Dialyselösung ohne Zusatz von Citrat oder Phosphat, mit Zusatz von Citrat und mit Zusatz von Phosphat.

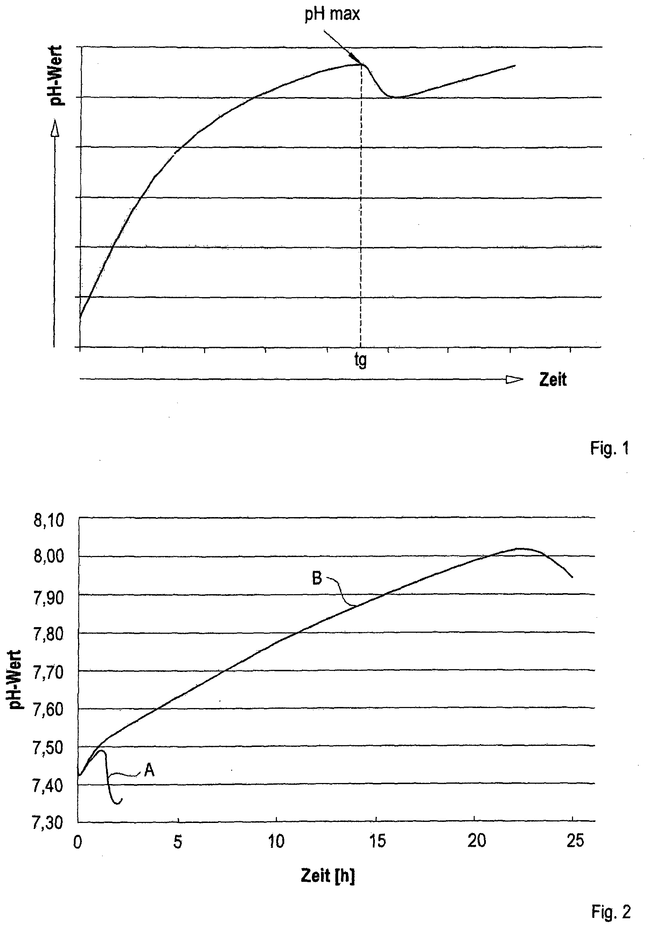

Figur 1 zeigt den zeitlichen Verlauf des pH-Wertes einer Dialyselösung über die Zeit während der Ausgasung von CO₂ aus der Dialyselösung.

Zur Bestimmung der Stabilität der Dialyselösung kann beispielsweise die "Rapid Controlled Precipitation Method" oder die "Critical pH-method" herangezogen werden, wie sie in F. Hui et al: Journal European of Water Quality (Journal Europeen d'Hydrologie) T.33 Fasc. 1 (2002) beschrieben ist.

Die im Rahmen dieser Erfindung beschriebenen Ergebnisse wurden durch eine modifizierte Rapid Controlled Precipitation Method erhalten. Der Versuchsaufbau besteht aus sechs 3-Halskolben (Carousel-6 der Fa. Radleys), die nach oben hin geöffnet sind, um eine gleichmäßige Ausgasung von CO₂ aus der Lösung zu gewährleisten. Weiterhin erlaubt dieser Aufbau eine in-line Messung von z.B. pH-Wert und Leitfähigkeit sowie das simultane Heizen der Kolben.

Das Grundprinzip der verwendeten Methode besteht darin, dass der pH-Wert der Mischlösung bzw. der Dialyselösung durch kontrollierte Ausgasung von CO₂ langsam angehoben wird, bis die Dialyselösung einen metastabilen Zustand erreicht. Dies ist in Figur 1 bis zu dem Zeitpunkt tg ersichtlich.

Kollabiert die Dialyselösung durch Ausfällung von Calciumcarbonat, ist dies durch einen Abfall des pH-Wertes und der Leitfähigkeit erfassbar. Zeitlich unmittelbar danach kann optisch ein weißer Niederschlag beobachtet werden. Der maximale pH-Wert, der in Figur 1 als "pH max" bezeichnet ist, bis zu dem keine Ausfällungen auftreten, wird als Kenngröße für die Stabilität einer Dialyselösung angesehen.

Wie ausgeführt ist der Zeitpunkt tg (time of germination) der erste Messpunkt, bei dem ein Abfall im pH-Wert detektiert wird.

In Figur 1 lässt sich die Erhöhung des pH-Wertes bis zum Zeitpunkt tg durch das Ausgasen von CO₂ aus der Dialyselösung erklären. Wie dies weiter aus Figur 1 ersichtlich ist, entsteht ein lokales pH-Wert Maximum. Nach diesem Punkt tritt Übersättigung der Dialyselösung ein und es kommt zur Ausfällung von Calciumcarbonat. Bei der Ausfällung werden der Dialyselösung Carbonat-Ionen entzogen. Der pH-Wert sinkt und es werden aufgrund der Gleichgewichtsreaktion mit Hydrogencarbonat vermehrt Protonen gebildet, was zu der Abnahme des pH-Wertes führt.

Durch den Zusatz von Phosphat bzw. von Orthophosphat kann die Stabilität der Dialyselösung oder einer Einzellösung signifikant erhöht werden, wobei das Zusammenbrechen des metastabilen Bereiches verzögert oder ganz verhindert wird.

Dies ergibt sich aus Figur 2. Diese Figur zeigt als Linie A den pH-Wert Verlauf einer Dialyselösung enthaltend Natrium, Calcium, Magnesium, Chlorid, Hydrogencarbonat und Glucose, jedoch ohne Phosphat, über die Zeit. Linie B zeigt den pH-Wert Verlauf einer identischen Dialyselösung, die als einzigen Unterschied 0,1 mmol/l Phosphat enthält.

Der der Figur 2 zugrundeliegende Versuch wurde mit einem Füllvolumen von 275 ml durchgeführt und die 3-Halskolben wurden mit Blenden mit Öffnungen von 3 mm Durchmesser verschlossen, um die Ausgasung von CO₂ zu kontrollieren. Der Versuch wurde mit einem Start-pH-Wert von 7,4 und bei einer Temperatur von 60 °C durchgeführt.

Wie dies aus dem Verlauf gemäß Linie A hervorgeht, nimmt der pH-Wert zunächst bis zu einem Maximalwert von 7,47 zu. Sodann nimmt der pH-Wert aufgrund der Ausfällung von Calciumcarbonat ab. Dies findet zu einem Zeitpunkt von 45 min nach Versuchsbeginn statt, d.h. tg = 45 min.

Linie B zeigt, dass die Zugabe von Phosphat einen wesentlichen Einfluss auf den pH-Wert Verlauf hat. Der maximal erreichbare pH-Wert beträgt 8,02 und die Zeitdauer bis zum Eintreten der Ausfällung von Calciumcarbonat beträgt ca. 22 Stunden. Dies bedeutet, dass durch die Anwesenheit von Phosphat nicht nur der pH-Wert, bei dem eine Calciumcarbonat-Ausfällung eintritt, sondern auch die Zeitspanne, bis diese Ausfällung eintritt, erhöht ist.

Die Wirkung von Phosphat als die Dialyselösung stabilisierendes Mittel ist temperatur- und konzentrationsabhängig.

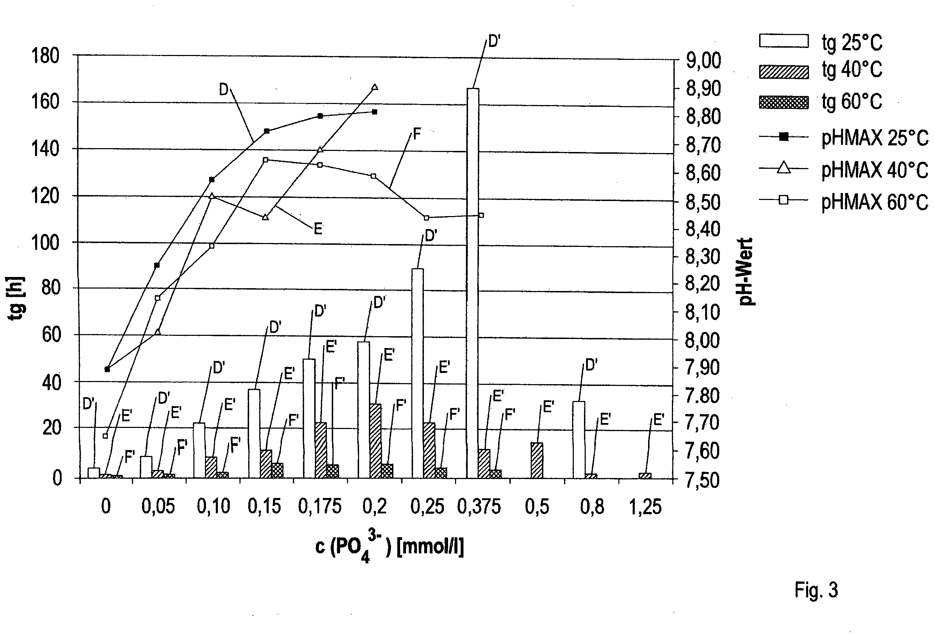

Figur 3 zeigt die Verläufe des maximalen pH-Wertes, d.h. des pH-Wertes, der bei dem Eintreten der Ausfällung gemessen wird, sowie die Zeitspanne (tg), die ab Versuchsbeginn bis zur Ausfällung verstreicht. Die Versuche wurden mit dem zu Figur 1 beschriebenen Versuchs-Setup durchgeführt.

Wie aus Figur 3 ersichtlich, sind die stabilisierenden Effekte von Orthophosphat von der Temperatur und von der Konzentration abhängig. Die Stabilität der Dialyselösungen hängt signifikant von der Phosphatkonzentration ab, was sich sowohl in den jeweils erreichten pH-max Werten, d.h. in den maximalen pH-Werten ausdrückt, bis zu denen die Dialyselösungen stabil sind, als auch in den tg-Werten, d.h. in den Zeitspannen, die ab dem Versuchsbeginn vergehen, bis die Ausfällung eintritt und der pH-Wert wieder absinkt.

In Figur 3 sind die Linien D, E und F die Verläufe der pH-max Werte für eine Lösungstemperatur von 25 °C (Linie D), 40 °C (Linie E) und 60 °C (Linie F).

Aus Figur 3 ergibt sich des Weiteren deutlich eine Temperaturabhängigkeit des Stabilisierungseffektes. So ist aus Figur 3 erkennbar, dass beispielsweise bei einer Temperatur von 25 °C (Säulen D') eine Dialyselösung mit einem Gehalt an Phosphat 0,375 mmol/l die größte Stabilität aufweist, bei einer Temperatur von 40 °C (Säulen E') jedoch eine Dialyselösung mit einem Phosphat-Gehalt von 0,2 mmol/l am stabilsten ist. Bei einer Temperatur von 60 °C (Säulen F') zeigt eine Dialyselösung mit einer Phosphat-Konzentration von 0,15 mmol/l die größte Stabilität.

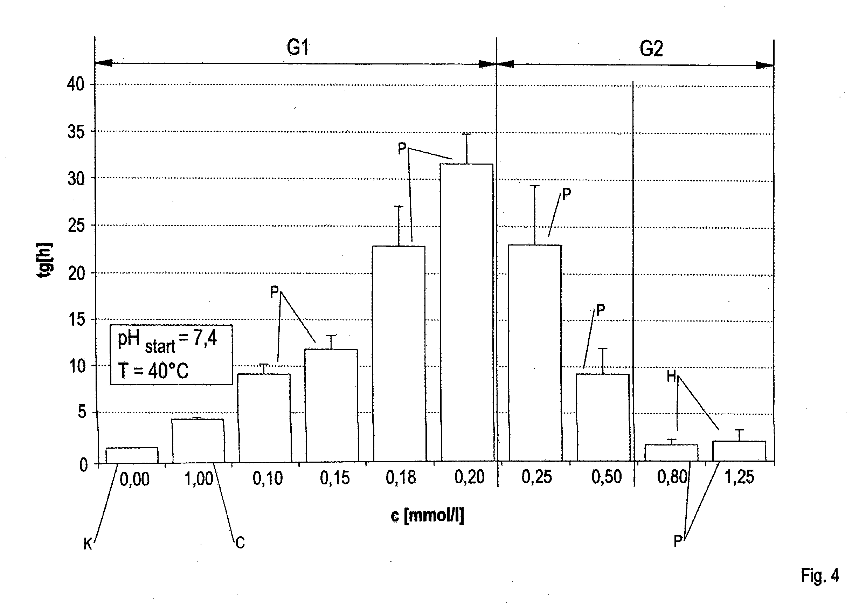

Figur 4 zeigt die Konzentrationsabhängigkeit des stabilisierenden Effektes exemplarisch für eine Lösungstemperatur von 40 °C.

Wie aus Figur 4 ersichtlich und übereinstimmend mit Figur 3 wird bei dieser Temperatur ein Stabilitätsmaximum bei einer Phosphatkonzentration von 0,2 mmol/l erreicht. Die phosphathaltigen Dialyselösungen sind mit dem Buchstaben P markiert. Bei dieser Temperatur nimmt bei einer weiteren Erhöhung der Phosphatkonzentration die Stabilität der Dialyselösung wieder ab, was dadurch erkennbar ist, dass die Zeitspanne tg bis zur Ausfällung wieder kleiner wird.

Die Abnahme der Stabilität der Dialyselösung bei der genannten Temperatur von 40 °C ab einer Phosphat-Konzentration von 0,2 mmol/l ist darauf zurückzuführen, dass schwerlösliche Calciumphosphate gebildet werden.

In dem Bereich G1 ist keine Calciumphosphat Ausfällung zu beobachten, hingegen tritt in dem Bereich G2 eine Calciumphosphat Ausfällung auf.

Aus Figur 4 ist weiter ersichtlich, dass eine Zugabe von Citrat (c = 1 mmol/l; kein Phosphat) (Buchstabe C) zwar eine gewisse Stabilisierung der Dialyselösung im Vergleich zu einer Dialyselösung ohne Stabilisierungsmittel (Buchstabe K) bewirkt, dass jedoch der stabilisierende Effekt im Falle von Phosphat wesentlich ausgeprägter ist.

So ist eine Dialyselösung mit 0,1 mmol/l Phosphat unter den oben genannten Versuchsbedingungen ca. zweifach länger stabil als eine Dialyselösung mit 1 mmol/l Citrat.

Weiter ergibt sich aus Figur 4, dass eine Konzentration von Phosphat im medizinisch relevanten Bereich von 0,8 mmol/l bis 1,25 mmol/l (Buchstaben H) bei sonst analoger Lösungszusammensetzung keine erhöhte Stabilität aufweisen.

Zusammenfassend lässt sich somit festhalten, dass die Zugabe von Phosphat bzw. von Orthophosphat in den beanspruchten Konzentrationsbereichen zur einer signifikanten Erhöhung der Stabilität Calcium-haltiger, bicarbonatgepufferter Dialyselösungen führt. Die Wahrscheinlichkeit von Ausfällungsreaktionen kann wesentlich reduziert werden, was die Sicherheit wie auch die Haltbarkeit von Dialyselösungen deutlich verbessert, ohne die medizinische Wirksamkeit zu beeinflussen. Die erfindungsgemäß geringen Phosphatkonzentrationen haben keine medizinische Wirksamkeit, so dass die Dialyselösungen ohne weiteres im Rahmen der Dialyse eingesetzt werden können.

## Patentansprüche

1. Kombination aus mehreren, vorzugsweise aus genau zwei Einzellösungen, die derart ausgebildet sind, dass sie nach ihrem Mischen miteinander eine Dialyselösung enthaltend Bicarbonat, Calcium sowie Phosphat ausbilden, wobei die Dialyselösung Phosphat mit einer Konzentration im Bereich von bis zu 0,4 mmol/l, vorzugsweise im Bereich von bis zu 0,375 mmol/ oder im Bereich von bis zu 0,25 mmol/l enthält und besonders bevorzugt im Bereich von bis zu 0,2 mmol/l enthält, wobei eine erste Einzellösung Calcium, Magnesium, Chlorid, ggf. Glucose und ggf. Kalium enthält und eine zweite Einzellösung Natrium, Chlorid, Hydrogencarbonat und Phosphat enthält,
**dadurch gekennzeichnet,**
**dass** die erste Einzellösung kein Natrium enthält, dass die erste Einzellösung einen pH-Wert im Bereich von 2,4 bis 3,0 und die zweite Einzellösung einen pH-Wert im Bereich von 7,0 bis 7,8 aufweist, und dass die zweite Einzellösung in einem größeren Volumen als die erste Einzellösung vorliegt.

2. Kombination nach Anspruch 1, **dadurch gekennzeichnet, dass** die Dialyselösung Phosphat in einem Bereich von 0,05 mmol/l bis 0,25 mmol/l enthält.

3. Kombination nach Anspruch 2, **dadurch gekennzeichnet, dass** die Dialyselösung Phosphat in einem Bereich von zumindest 0,05 mmol/l aufweist.

4. Kombination nach einem der vorhergehenden Ansprüche, **dadurch gekennzeichnet, dass** die erste Einzellösung kein Hydrogencarbonat und/oder kein Phosphat enthält.

5. Kombination nach einem der vorhergehenden Ansprüche, **dadurch gekennzeichnet, dass** die zweite Einzellösung kein Calcium und/oder kein Magnesium und/oder kein Kalium und/oder keine Glucose enthält.

6. Mehrkammerbeutel umfassend wenigstens zwei Kammern, wobei eine der Kammern die erste Einzellösung der Kombination und eine andere Kammer die zweite Einzellösung der Kombination gemäß einem der vorhergehenden Ansprüche aufweist.

7. Mehrkammerbeutel nach Anspruch 6, **dadurch gekennzeichnet, dass** der Mehrkammerbeutel wenigstens eine Naht oder ein sonstiges Trennmittel aufweist, die zwei Kammern voneinander trennt, wobei die Naht oder das Trennmittel vorzugsweise durch Druck auf eine der Kammern geöffnet werden kann.

## Claims

1. A combination of a plurality of, preferably exactly two individual solutions which are configured such that they form a dialysis solution after their mixing with one another containing bicarbonate, calcium and phosphate, wherein the dialysis solution contains phosphate having a concentration in the range of up to 0.4 mmol/l, preferably in the range of up to 0.375 mmol/l, or in the range of up to 0.25 mmol/l, and particularly preferably in the range of up to 0.2 mmol/l, wherein a first individual solution contains calcium, magnesium, chloride, optionally glucose and optionally potassium and a second individual solution contains sodium, chloride, hydrogen carbonate and phosphate,
**characterized in that**
the first individual solution does not contain any sodium; **in that** the first individual solution has a pH in the range from 2.4 to 3.0 and the second individual solution has a pH in the range from 7.0 to 7.8; and **in that** the second individual solution is present in a larger volume than the first individual solution.

2. A combination in accordance with claim 1, **characterized in that** the dialysis solution contains phosphate in a range from 0.05 mmol/l to 0.25 mmol/l.

3. A combination in accordance with claim 2, **characterized in that** the dialysis solution comprises phosphate in a range from at least 0.05 mmol/l.

4. A combination in accordance with one of the preceding claims, **characterized in that** the first individual solution does not contain any hydrogen carbonate and/or any phosphate.

5. A combination in accordance with one of the preceding claims, **characterized in that** the second individual solutions does not contain any calcium and/or any magnesium and/or any potassium and/or any glucose.

6. A multi-chamber bag comprising at least two chambers, wherein one of the chambers has the first individual solution of the combination in accordance with one of the preceding claims and another chamber has the second individual solution of the combination in accordance with one of the preceding claims.

7. A multi-chamber bag in accordance with claim 6, **characterized in that** the multi-chamber bag has at least one seam or another separating means which separates two chambers from one another, wherein the seam or the separating means can preferably be opened by pressure on one of the chambers.

## Revendications

1. Combinaison de plusieurs, de préférence exactement deux, solutions individuelles, qui sont conçues de telle manière qu'après leur mélange, elles forment une solution de dialyse contenant du bicarbonate, du calcium et du phosphate, la solution de dialyse contenant du phosphate dans une concentration allant jusqu'à 0,4 mmol/l, de préférence allant jusqu'à 0,375 mmol/l ou allant jusqu'à 0,25 mmol/l et de manière particulièrement préférée allant jusqu'à 0,2 mmol/l, une première solution individuelle contenant du calcium, du magnésium, du chlorure, le cas échéant du glucose et le cas échéant du potassium et une seconde solution individuelle contenant du sodium, du chlorure, de l'hydrogénocarbonate et du phosphate,
**caractérisée en ce que**
la première solution individuelle ne contient pas de sodium, **en ce que** la première solution individuelle présente une valeur de pH comprise dans la plage de 2,4 à 3,0 et la seconde solution individuelle présente une valeur de pH comprise dans la plage de 7,0 à 7,8, et **en ce que** le volume de la seconde solution individuelle est supérieur à celui de la première solution individuelle.

2. Combinaison selon la revendication 1, **caractérisée en ce que** la solution de dialyse contient de 0,05 mmol/l à 0,25 mmol/l de phosphate.

3. Combinaison selon la revendication 2, **caractérisée en ce que** la solution de dialyse contient au moins 0,05 mmol/l de phosphate.

4. Combinaison selon l'une des revendications précédentes, **caractérisée en ce que** la première solution individuelle ne contient pas d'hydrogénocarbonate et/ou pas de phosphate.

5. Combinaison selon l'une des revendications précédentes, **caractérisée en ce que** la seconde solution individuelle ne contient pas de calcium et/ou pas de magnésium et/ou pas de potassium et/ou pas de glucose.

6. Poche à compartiments multiples comprenant au moins deux compartiments, l'un des compartiments comportant la première solution individuelle de la combinaison et un autre compartiment la seconde solution individuelle de la combinaison selon l'une des revendications précédentes.

7. Poche à compartiments multiples selon la revendication 6, **caractérisée en ce que** la poche à compartiments multiples comporte au moins une soudure ou un autre moyen de séparation, qui sépare deux compartiments l'un de l'autre, la soudure ou l'autre moyen de séparation pouvant être ouvert(e) de préférence par une pression sur un des compartiments.
